# EUROPÄISCHE PATENTSCHRIFT

(11) **EP 1 024 762 B1**
(45) Veröffentlichungstag und Bekanntmachung des Hinweises auf die Patenterteilung: **30.07.2003**
(21) Anmeldenummer: 97943713.4
(22) Anmeldetag: 20.10.1997
(51) Int. Cl.: A61B 17/72

(54) **KNOCHENFIXATIONSVORRICHTUNG**
BONE FIXATION DEVICE
DISPOSITIF D'OSTEOSYNTHESE

(43) Veröffentlichungstag der Anmeldung: 09.08.2000
(73) Patentinhaber: SYNTHES AG Chur, 7002 Chur (CH)
(72) Erfinder: LEU, Dieter, CH-4613 Rickenbach (CH); DÄSCHER, Peter, CH-7270 Davos Platz (CH)
(74) Vertreter: Lusuardi, Werther Giovanni, Dr.
(86) Internationale Anmeldenummer: CH9700392
(87) Internationale Veröffentlichungsnummer: WO99020195

(56) Entgegenhaltungen:
- EP-A- 0 447 824
- WO-A-94/13219
- WO-A-95/26688
- DE-C- 4 341 677
- US-A- 5 122 141
- US-A- 5 531 748

## Beschreibung

Die Erfindung betrifft eine Vorrichtung zur Fixation von Knochenfrakturen gemäss dem Oberbegriff des Patentanspruchs 1.

Am gebrochenen Tibiakopf gilt es eine lasttragende, frakturierte Fläche zu rekonstruieren. Die Tibia ist der einzige Knochen des Körpers, welcher kontinuierlich von einem kubischen Knochen mit einer lasttragenden Gelenkfläche in einen röhrenförmigen Knochen übergeht. Im Bereich des Tibiakopfes werden die auf die proximalen Tibiagelenkflächen axial einwirkenden Belastungskräfte kontinuierlich in eine Belastung eines Röhrenknochens überführt. Spezielle Implantate für diese Verhältnisse existieren noch nicht. Ein idealer Lastträger für den Tibiakopf muss diesen speziellen Verhältnissen gerecht werden.

Der Stand der Technik kennt verschiedene gerade Platten und Winkelplatten sowie Marknägel zur Behandlung von Tibiakopffrakturen. Alle diese bekannten Implantate sind jedoch auf den jungen, starken Knochen ausgerichtet, galt es doch bislang vorallem unfallbedinge Frakturen (Sport-, Arbeits- und Verkehrsunfälle) zu versorgen. Bedingt durch das zunehmende Alter der Bevölkerung und auch der Aktivität der älteren Bevölkerung müssen nun aber in zunehmendem Masse auch Frakturen porotischer Knochen versorgt werden.

Die Osteoporose stellt ein zunehmendes Problem im Gesundheitswesen dar. Der Anteil der über 80-Jährigen nimmt markant zu, so dass auch die Frakturen bei Osteoporose deutlich zunehmen; Frakturen der proximalen und distalen Tibia, des proximalen und distalen Femurs, aber auch des proximalen Humerus und des distalen Vorderarmes stehen dabei im Vordergrund.

Die bisherigen osteosynthetischen Implantate, welche zum Stand der Technik gehören, sind zur Anwendung am porotischen Knochen, mit seiner dünnen Kortikalis und seiner qualitativ schlechteren Spongiosa, nur wenig geeignet. Die korrekte Reposition ist bei einer dünnen Kortikalis oft erschwert. Die Fixation, respektive das Festhalten der reponierten Fraktur bietet im porotischen Knochen oft erhebliche Probleme, da die ossäre Verankerung der Implantate erschwert ist.

Zur Behandlung von proximalen Tibiafrakturen und speziell der Tibiakopffrakturen sind aber weder für die jungen starken, noch für porotische Knochen wirklich optimierte Implantate vorhanden. Es existieren lediglich verschiedene, leicht modifizierte Platten und Marknägel.

Alle Platten werden nach der Reposition der Fraktur mit Schrauben an den Knochen fixiert. Als nicht axiale Implantate sind diese Plattensysteme immer einer gewissen Biegebelastung ausgesetzt. Ein winkelstabile Fixation, welche bei Gelenksfrakturen oder bei gelenksnahen Frakturen benötigt wird, ist mit Platten und Schrauben nicht oder nur sehr bedingt zu erreichen. Marknägel können für Gelenksfrakturen nicht und für gelenksnahe Frakturen nur bedingt eingesetzt werden.

Aus der WO94/13219 ist ein modularer Marknagel bekannt, welcher aus einem Marknagel und einer über sein proximales Ende teleskopisch aufschiebbaren Hülse besteht. Die Hülse bleibt axial gleitbar, damit das Knochenfixationsmittel sowohl durch Öffnungen in der Hülse als auch durch eine im Marknagel vorhandenes Langloch hindurchgeführt werden kann. Eine sichere Fixation zwischen Marknagel und Hülse kann damit nicht erreicht werden. Das Knochenfixationsmittel kann in der Hülse nicht winkelstabil verschraubt werden.

Aus der EP-B 544.868 ist eine Knochenschraube mit gewindetragendem Schraubenkopf bekannt, wobei die Steigung des Schraubenkopfgewindes kleiner ist als diejenige des Schraubenschaft-Gewindes. Diese Differentialschraube ist zum direkten Einschrauben in einen frakturierten Knochen vorgesehen; eine Verwendung bei welcher der Schraubenkopf in ein Implantat eingeschraubt würde fehlt vollständig.

Aus der EP 0 118 778 ist ein Verriegelungsnagel mit einem länglichen hohlen Körper bekannt, welcher am vorderen Ende abgerundet ist und ein Einschlagende mit einer Erweiterung aufweist. Zudem sind mindestens zwei Querbohrungen im Körper zur Aufnahme jeweils einer Knochenschraube vorgesehen. Das Querschnittsprofil des Körpers ist über seinen Umfang ringförmig geschlossen.

Ein weiterer, aus Vollmaterial bestehender Verriegelungsnagel ist in der EP 0 447 824 FRIGG offenbart. Hier weist der proximale Endteil eine Querschnittsfläche auf, welche sowohl in der anterioren, als auch in der posterioren Hälfte trigonal ausgebildet ist und gesamthaft annähernd quadratisch ist. Der distale Endteil weist eine Querschnittsfläche auf, welche in der anterioren Hälfte annähernd trigonal und in der posterioren Hälfte annähernd semisphärisch ist. Diese spezielle Konstruktion des erfindungsgemässen Marknagels bewirkt einerseits eine hohe Rotationsstabilität im proximalen spongiösen Teil der Tibia und andererseits eine möglichst optimale Anpassung an die Geometrie der Markraumhöhlung im distalen, kortikalen Teil der Tibia.

Wiederum ein weiterer Verriegelungsnagel ist aus der DE 43 41 677 SCHROEDER bekannt. Der Nagelkörper ist am vorderen distalen Ende abgerundet und weist ein proximales Einschlagende auf. Im Nagelkörper sind Querbohrungen zur Aufnahme jeweils einer Knochenschraube angeordnet. Zudem besteht der Nagelkörper aus Vollmaterial und weist zumindest an einer Seite jeder Querbohrung eine trichterförmige Öffnung auf.

Hier will die Erfindung Abhilfe schaffen. Der Erfindung liegt das Problem zugrunde, ein Vorrichtung zur Fixation von Knochen frakturen zu schaffen, mit welcher folgende Frakturen auch bei Osteoporose optimal behandelt werden können:
1. Tibiaschaftfrakturen:
   - Frakturen des proximalen Tibiaschaftes
   - ipsylaterale Tibiakopffrakturen
2. Tibiakopffrakturen
   - Frakturen des medialen und lateralen Tibiaplateaus
   - Frakturen der dorsalen Gelenkanteile der proximalen Tibia
   - ipsylaterale Tibiaschaftfrakturen
3. analoge Frakturen beim distalen oder proximalen Femur (und dabei folgende Bedingungen erfüllen):
   - sie bietet eine stabile Fixation, auch bei Osteoporose, durch eine biomechanische optimale (axiale) Lage, und eine winkelstabile Fixation sowie eine grosse Auflagefläche zwischen Knochen und Implantat
   - sie ist einfach und sicher in der Handhabung (z.B. durch Vermeidung eines hinteren Zuganges bei dorso-proximalen Tibiakopffrakturen);
   - sie ist für die linke und rechte Tibia, sowie für das mediale und laterale Tibiaplateau anwendbar.

Ferner liegt der Erfindung das Problem zugrunde, mittels einer quer zur Marknagelachse verlaufenden Knochenschraube ein Knochenfragment zu erfassen und dieses gegen den Marknagel ziehen zu können, wodurch ein intrafragmentärer Druck erzeugbar ist.

Die Erfindung löst generell die gestellte Aufgabe mit einer Vorrichtung, welche die Merkmale des Anspruchs 1 aufweist.

Ein Marknagel im Tibiakopf liegt als axiales Implantat biomechanisch in optimaler Lage. Mittels Bolzen wird der Marknagel im Markraum der Tibia fixiert. Wird der Marknagel zusätzlich an die ventrale Kortikalis gezogen, so vergrössert sich die Auflagefläche zwischen porotischem Knochen und Implantat, die Kontaktkräfte werden dadurch verringert. Mit Schrauben, welche winkelstabil im Marknagel verschraubt sind, kann der Tibiakopf rekonstruiert werden. Der Marknagel mit den winkelstabil verschraubten Tibiakopfschrauben übernimmt biomechanisch die Funktion einer intramedullären Winkelplatte. Damit sind die Vorteile eines axialen Implantates (Marknagel) mit den Vorteilen eines winkelstabilen Implantates (Winkelplatte) kombiniert.

Beim erfindungsgemässen Implantat wird im Gegensatz zu den bekannten Osteosynthese-Systemen der Lastträger vorerst in biomechanischer Lage eingebracht und im intakten Knochen verankert. Die Fraktur wird anschliessend an diesen fest verankerten Lastträger reponiert und winkelstabil fixiert. Da die winkelstabile Frakturfixation im fest verankerten Lastträger erfolgt, ist die Knochenqualität (Porose) von sekundärer Bedeutung.

Eine bevorzugte Weiterbildung der Erfindung besteht darin, dass der Marknagel sowohl im Bereich des distalen als auch des proximalen Endes mindestens je eine quer zur Zentralachse verlaufende Bohrung aufweist, um den Marknagel verriegeln zu können.

Der Marknagelkopf weist eine, vorteilhafterweise mindestens zwei oder mehr Bohrungen auf, deren Achsen parallel oder divergierend sein können und welche ein Gewinde aufweisen können.

Das im Marknagelkopf einzuführende Knochenfixationsmittel ist vorzugsweise eine Knochenschraube mit einem Schraubenkopf und einem Schraubenschaft-Gewinde, wobei die Knochenschraube vorzugsweise einen zentral durchgehenden Kanal aufweist. Das schraubenkopfnahe Gewinde weist vorzugsweise eine kleinere Steigung auf als das Gewinde des Schraubenschaftes. Vorzugsweise ist zwischen den beiden differentiellen Gewinden ein gewindeloser Schaftabschnitt vorgesehen. Damit ist es möglich ein mit dieser Knochenschraube erfasstes Knochenfragment heranzuziehen, um einen intrafragmentären Druck zu erzeugen.

Die Spitze dieser Knochenschraube ist vorzugsweise selbstbohrend selbstschneidend ausgebildet. Dadurch lässt sich der Arbeitsablauf vereinfachen.

Statt der Knochenschrauben können auch eine Klinge oder eine Platte als Knochenfixationsmittel verwendet werden. Die Klinge oder die Platte ist vorzugsweise geschlitzt oder gewindetragend durchbohrt, um Bolzen oder Schrauben aufnehmen zu können. Damit lässt sich eine winkelstabile, intramedulläre Verschraubung erreichen.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch die im Knochen implantierte erfindungsgemässe Vorrichtung mit einem Marknagelkopf für Tibiakopfschrauben;
Fig. 2 eine vergrösserte perspektivische Darstellung des Marknagelkopfes von Fig. 1 versetzt um 90°;
Fig. 3 eine vergrösserte perspektivische Darstellung der Tibiakopfschraube von Fig. 1;
Fig. 4 eine perspektivische Darstellung einer im Knochen implantierten erfindungsgemässen Vorrichtung mit einem Marknagelkopf für eine intramedulläre Tibiakopfkreuzplatte;
Fig. 5 eine perspektivische Darstellung einer Marknagelkopfes mit Kreuzschlitz;
Fig. 6 eine perspektivische Darstellung einer im Knochen implantierten erfindungsgemässen Vorrichtung nach Fig. 4 im Endzustand; und
Fig. 7 eine perspektivische Darstellung einer Tibiakopf-Knochenplatte für eine Variante der Vorrichtung nach Fig. 4.

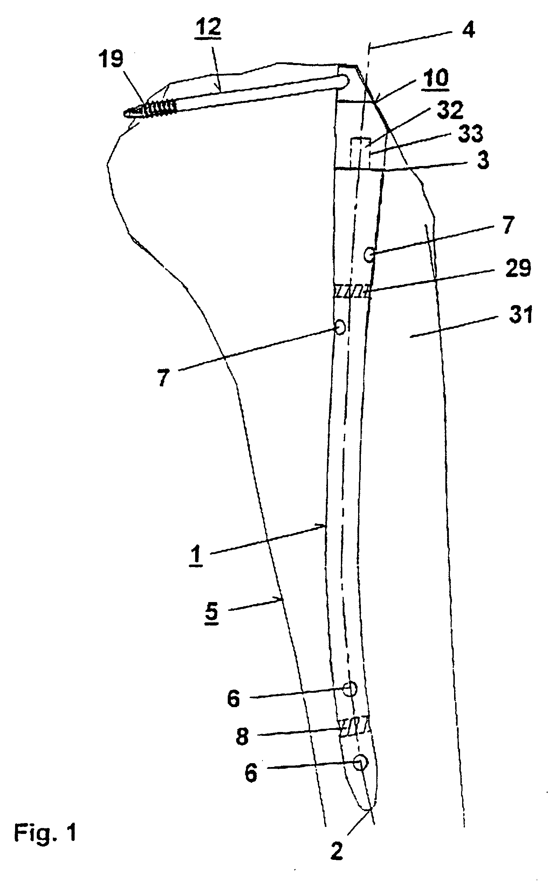

Die in Fig. 1 dargestellte erfindungsgemässe Vorrichtung umfasst einen bekannten Marknagel 1 mit einem distalen Ende 2, einem proximalen Ende 3 und einer Zentralachse 4. Der Marknagel 1 ist leicht gekrümmt und das distale Ende 2 ist stumpf ausgebildet. Im Bereich des distalen Endes 2 sind zwei quer zur Zentralachse 4 verlaufende Bohrung 6 angebracht, in welchen Verriegelungsbolzen eingeführt werden können. Zwischen den beiden Bohrungen 6 ist eine zusätzliche in antero-posteriorer Richtung (in etwa der Zeichenebene entsprechend) verlaufende, gewindetragende Bohrung 8 vorgesehen, welche zur genauen Positionierung des Marknagels 1 im Markraum des Knochens 5 dient.

Im Bereich des proximalen Endes 3 sind zwei quer zur Zentralachse 4 verlaufende Bohrungen 7 vorgesehen, in welche weitere Verriegelungsbolzen eingeführt werden können. Mit einer dritten gewindetragenden Bohrung 29 in antero-posteriorer Richtung kann der Marknagel an die ventrale Kortikalis 31 gezogen werden. Dies erlaubt eine genaue Positionierung des Marknagels 1 im Markraum.

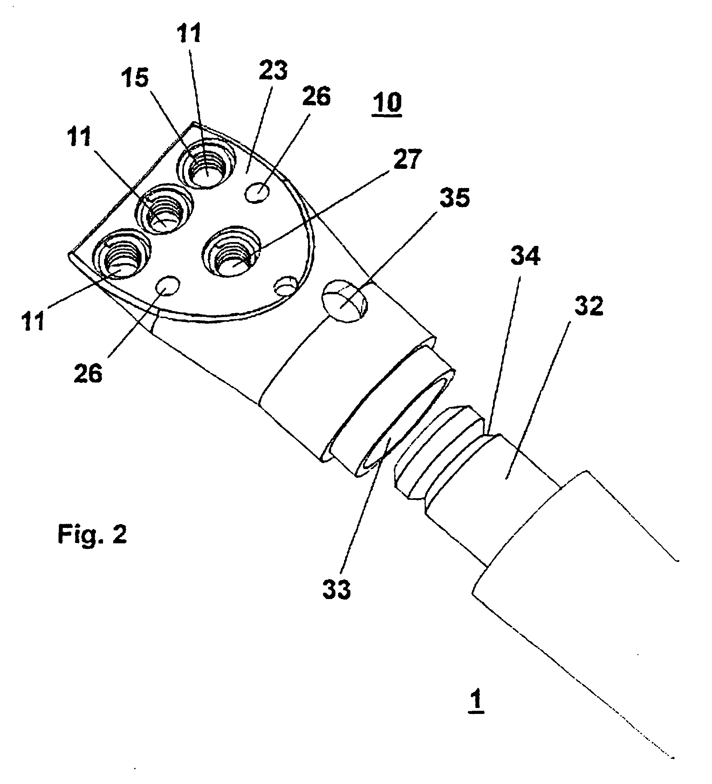

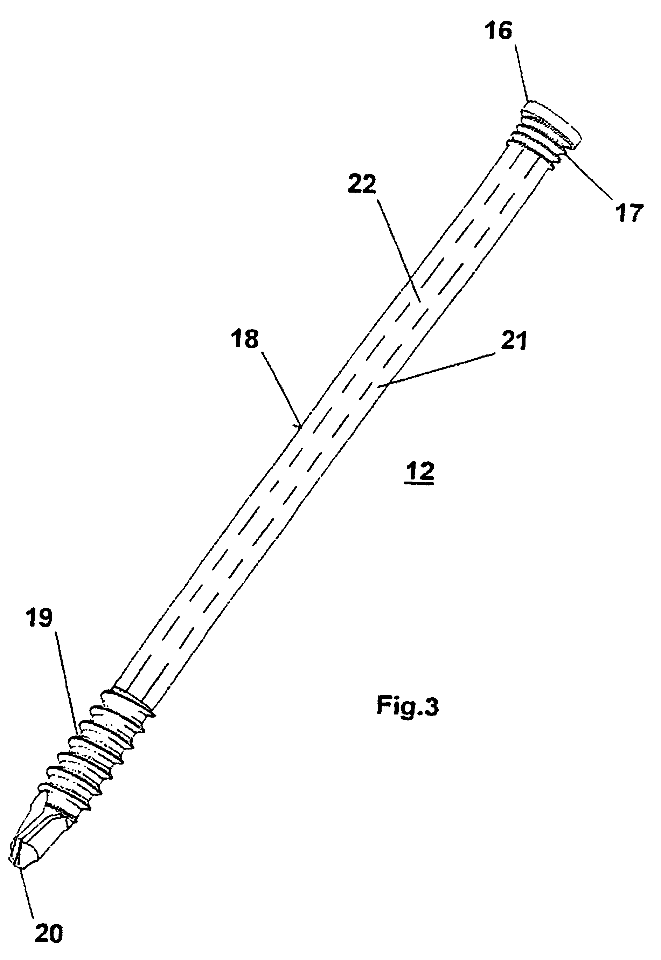

Wie in Fig. 2 näher dargestellt hat der Marknagelkopf 10 die Gestalt eines schief geschnittenen Zylinders, wobei die schiefe Fläche 23 von drei nebeneinanderliegenden Bohrungen 11 durchstossen wird, welche parallele oder divergierende Achsen aufweisen können. Die Bohrungen 11 des Marknagelkopfes 10 weisen alle ein Gewinde 15 auf, um Knochenschrauben 12 (Fig. 3) aufnehmen zu können, welche einen Schraubenkopf 16 mit einem entsprechendem Gewinde 17 aufweisen. Die Knochenschrauben 12 weisen einen Schraubenschaft 18 mit zwei Gewindeabschnitten 19 und 17 auf. Die Steigung des Gewindes 19 an der Schraubenspitze 20 ist grösser als das schraubenkopfnahe Gewinde 17. Die Knochenschrauben 12 weisen weiter einen zentral durchgehenden Kanal 22 auf, durch welchen Führungsdrähte geschoben werden können. Zwischen den beiden Gewinden 17;19 ist ein gewindeloser Schaftabschnitt 21 vorgesehen. Die Schraubenspitze 20 ist selbstbohrend und selbstschneidend ausgebildet.

Auf der schiefen Fläche 23 sind weitere Bohrungen 26,27 vorgesehen, um die üblichen Marknagel-Zielgeräte in lösbarer Weise daran befestigen zu können.

Der Marknagelkopf 10 kann - wie in Fig. 1 und 2 dargestellt mit seiner zylindrischen Bohrung 33 auf einen entsprechenden zylinderförmigen Zapfen 32 am proximalen Ende 3 des Marknagels 1 aufgesetzt werden. Der Zapfen 32 kann Längsrillen oder -nuten 34 aufweisen, welche in korrespondierenden Elemente (Nuten, beziehungsweise Rillen) im Inneren der Bohrung 33 eingreifen, um die beiden Teile 1,10 gegen Verdrehung zu sichern. die definitive auch axiale Fixation der beiden Teile 1,10 kann durch Einführung einer Fixationsschraube in die Querbohrung 35 des Marknagelkopfes 10 erfolgen.

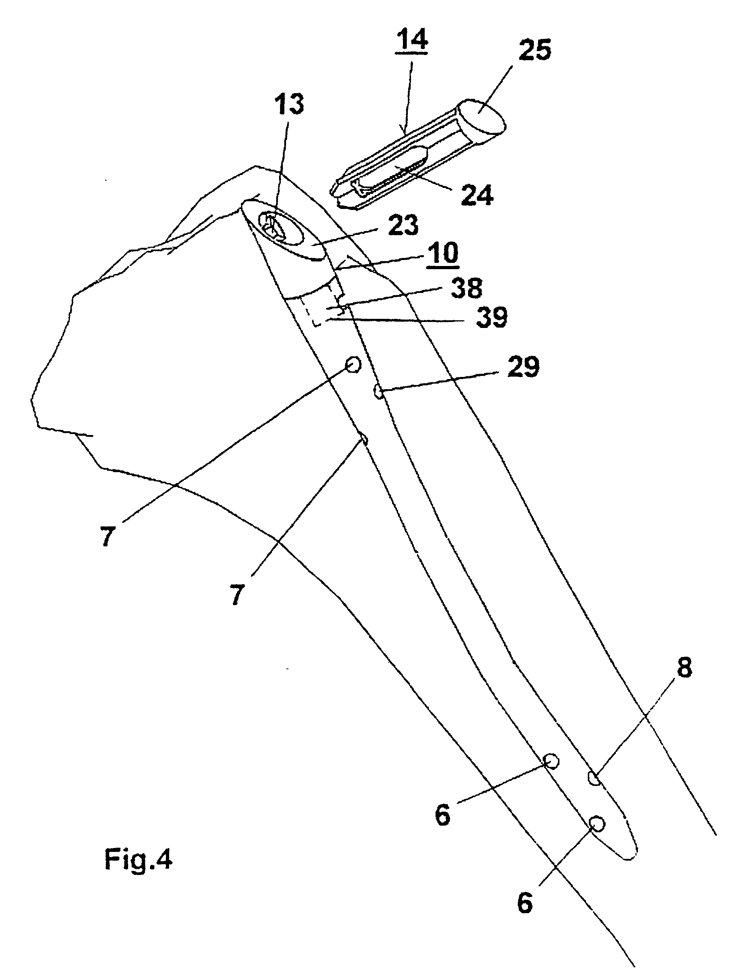

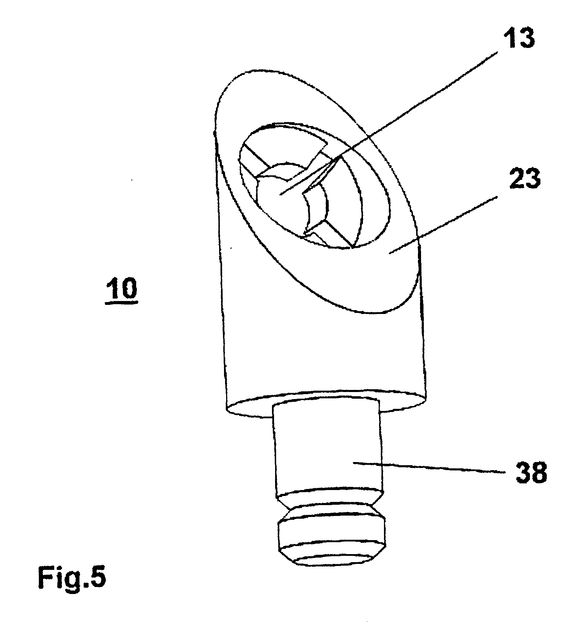

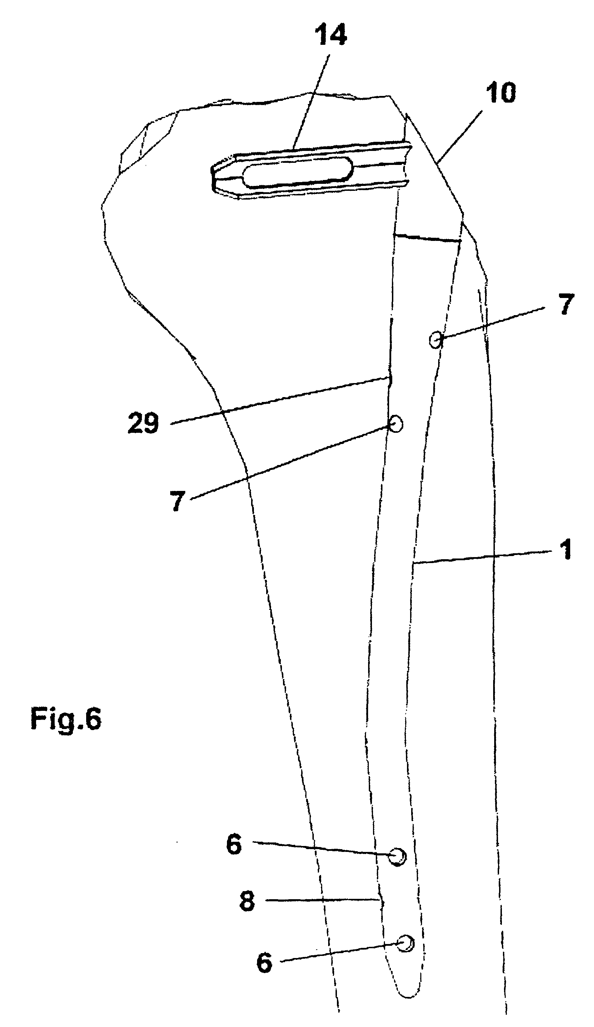

Bei einer weiteren in Fig. 4 dargestellten Ausführungsform der Erfindung ist als Knochenfixationsmittel statt der Knochenschraube 12 eine gekreuzte Klinge 14 vorgesehen, welche einen Schlitz 24 zur Aufnahme von Bolzen oder Schrauben besitzt. Zur Aufnahme dieser Klinge 14 ist der Marknagelkopf 10 gegenüber der Ausführung gemäss den Fig. 1 und 2 ebenfalls modifiziert. Wie in Fig. 5 dargestellt besitzt der Marknagelkopf 10 für diese Variante, statt der drei Bohrungen 11, einen einzigen Kreuzschlitz 13, der etwas vertieft in der schiefen Fläche 23 eingelassen ist. Eine weitere Modifikation bei dieser Ausführung des Marknagelkopfes 10 besteht darin, dass statt einer zylindrischen Bohrung 33 (Fig. 2) ein Zapfen 38 vorgesehen ist; dementsprechend weist der dazugehörige Marknagel 1 statt eines Zapfens eine Bohrung 39 auf.
Die gekreuzte Klinge 14 lässt in den Kreuzschlitz 13 einschieben bis ihr Kopf 25 mit der schiefen Fläche 23 bündig ist, so dass die Position wie in Fig. 6 dargestellt, erreicht wird.

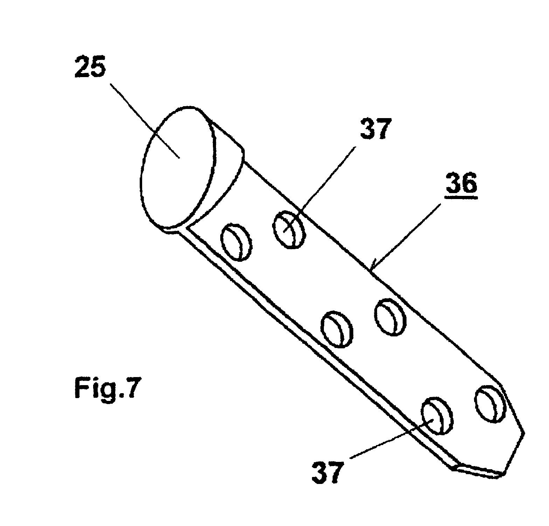

Statt der Klinge 14 kann auch eine - in Fig. 7 dargestellte - Knochenplatte 36 verwendet werden, welche gewindetragende Bohrungen 37 aufweist, welche in der Platte 36 verschieden angeordnet sein können. Der Kopf 25 ist gleich ausgebildet wie bei der Klinge 14, so dass eine formschlüssige und winkelstabile Einführung der Knochenplatte 36 in den Kreuzschlitz 13 (Fig. 4) garantiert ist.

## Patentansprüche

1. Vorrichtung zur Fixation von Knochenfrakturen, die einen Marknagel (1) umfasst mit einem distalen Ende (2), einem proximalen Ende (3) und einer Zentralachse (4), wobei
A) am proximalen Ende (3) des Marknagels (1) ein Marknagelkopf (10) vorgesehen ist, der mindestens eine quer zur Zentralachse (4) verlaufende Bohrung (11) oder einen Schlitz (13) zur winkelstabilen und formschlüssigen Aufnahme von damit korrespondierenden Knochenfixationsmitteln (12,14) aufweist; und
B) im Bereich des distalen Endes (2) mindestens eine gewindetragende Bohrung (8;29) vorgesehen ist,
**dadurch gekennzeichnet, dass**
C) die Vorrichtung zusätzlich als Knochenfixationsmittel (12;14) mindestens eine Knochenschraube (12) mit einem Schraubenkopf (16) und einem Schraubenschaft (18) mit Gewinde (19) umfasst; und
D) der Schraubenkopf (16) ein Gewinde (17) aufweist, welches eine kleinere Steigung aufweist als diejenige des Gewindes (19) des Schraubenschaftes (18).

2. Vorrichtung nach Anspruch 1, **dadurch gekennzeichnet, dass** der Marknagelkopf (10) als separates, auf das proximale Ende (3) des Marknagels (1) aufsetzbares und in lösbarer Weise daran fixierbares Element ausgebildet ist.

3. Vorrichtung nach Anspruch 1, **dadurch gekennzeichnet, dass** der Marknagelkopf (10) einstückig mit dem Marknagel (1) ausgebildet ist.

4. Vorrichtung nach einem der Ansprüche 1 bis 3, dass der Marknagel (1) im Bereich des distalen Endes (2) mindestens eine quer zur Zentralachse (4) verlaufende Bohrung (6) aufweist.

5. Vorrichtung nach einem der Ansprüche 1 bis 4, **dadurch gekennzeichnet, dass** der Marknagel (1) im Bereich des proximalen Endes (3) mindestens eine quer zur Zentralachse (4) verlaufende Bohrung (7) aufweist.

6. Vorrichtung nach einem der Ansprüche 1 bis 5, **dadurch gekennzeichnet, dass** der Marknagelkopf (10) mindestens drei Bohrungen (11) aufweist.

7. Vorrichtung nach Anspruch 6, **dadurch gekennzeichnet, dass** die Bohrungen (11) divergierende Achsen aufweisen.

8. Vorrichtung nach Anspruch 6, **dadurch gekennzeichnet, dass** die Bohrungen (11) parallele Achsen aufweisen.

9. Vorrichtung nach einem der Ansprüche 1 bis 8, **dadurch gekennzeichnet, dass** die Bohrung (11) des Marknagelkopfes (10) ein Gewinde (15) aufweist.

10. Vorrichtung nach Anspruch 1, **dadurch gekennzeichnet, dass** das Knochenfixationsmittel (12;14) eine Knochenschraube (12) mit einem Schraubenkopf (16) und einem Schraubenschaft (18) mit Gewinde (19) ist, wobei die Knochenschraube (12) vorzugsweise einen zentral durchgehenden Kanal (22) aufweist.

11. Vorrichtung nach Anspruch 1, **dadurch gekennzeichnet, dass** zwischen den beiden Gewinden (17;19) ein gewindeloser Schaftabschnitt (21) vorgesehen ist.

12. Vorrichtung nach einem der Ansprüche 1 bis 11, **dadurch gekennzeichnet, dass** die Spitze (20) der Knochenschraube (12) selbstbohrend oder selbstschneidend ausgebildet ist.

13. Vorrichtung nach einem der Ansprüche 1 bis 12, **dadurch gekennzeichnet, dass** das Knochenfixationsmittel (12;14) eine Klinge (14) oder Platte ist, welche vorzugsweise geschlitzt oder gewindetragend durchbohrt ist, zur Aufnahme von Bolzen oder Schrauben.

14. Vorrichtung nach einem der Ansprüche 1 bis 13, **dadurch gekennzeichnet, dass** eine weitere gewindetragende Bohrung (29) im Bereich des proximalen Endes (3) vorgesehen ist.

## Claims

1. Device for fixation of bone fractures comprising an intramedullary nail (1) with a distal end (2), a proximal end (3) and a central axis (4), whereby
A) an intramedullary nail head (10) is provided on the proximal end (3) of the intramedullary nail (1) having at least one bore (11) running across the central axis (4) or a recess (13) for form-fitting accommodation of corresponding bone fixation means (12, 14) at a stable angle; and
B) at least one threaded bore (8, 29) is provided in the area of the distal end (2),
**characterized in that**
C) the device also comprises at least one bone screw (12) with a screw head (16) and a screw shaft (18) with a thread (19) as bone fixation means (12, 14); and
D) the screw head (16) has a thread (17) which has a smaller pitch than that of the thread (19) of the screw shaft (18).

2. Device according to Claim 1, **characterized in that** the intramedullary nail head (10) is designed as a separate element which can be placed on the proximal end (3) of the intramedullary nail (1) and secured there in a detachable manner.

3. Device according to Claim 1, **characterized in that** the intramedullary nail head (10) is designed in one piece with the intramedullary nail (1).

4. Device according to one of Claims 1 through 3, **characterized in that** the intramedullary nail (1) has at least one bore (6) running across the central axis (4) in the area of the distal end (2).

5. Device according to one of Claims 1 through 4, **characterized in that** the intramedullary nail (1) has at least one bore (7) running across the central axis (4) in the area of the proximal end (3).

6. Device according to one of Claims 1 through 5, **characterized in that** the intramedullary nail head (10) has at least three bores (11).

7. Device according to Claim 6, **characterized in that** the bores (11) have divergent axes.

8. Device according to Claim 6, **characterized in that** the bores (11) have parallel axes.

9. Device according to one of Claims 1 through 8, **characterized in that** the bore (11) of the intramedullary nail head (10) has a thread (15).

10. Device according to Claim 1, **characterized in that** the bone fixation means (12, 14) comprises a bone screw (12) with a screw head (16) and a screw shaft (18) with a thread (19), where the bone screw (12) preferably has a continuous central channel (22).

11. Device according to Claim 1, **characterized in that** an unthreaded shaft section (21) is provided between the two threads (17, 19).

12. Device according to one of Claims 1 through 11, **characterized in that** the tip (20) of the bone screw (12) is designed to be self-tapping and self-cutting.

13. Device according to one of Claims 1 through 12, **characterized in that** the bone fixation means (12, 14) is a blade (14) or a plate having a preferably slotted or threaded bore to accommodate pins or screws.

14. Device according to one of Claims 1 through 13, **characterized in that** another threaded bore (20) is provided in the area of the proximal end (3).

## Revendications

1. Dispositif pour la fixation de fractures osseuses, qui comprend un clou médullaire (1) doté d'une extrémité distale (2), d'une extrémité proximale (3) et d'un axe central (4), dans lequel
A) à l'extrémité proximale (3) du clou médullaire (1) est prévue une tête (10) de clou médullaire qui présente au moins un alésage (11) ou une fente (13) qui s'étendent transversalement par rapport à l'axe central (4) pour recevoir de manière angulairement stable et en correspondance géométrique des moyens de fixation d'os (12, 14) qui y correspondent; et
B) dans la région de l'extrémité distale (2) est prévu au moins un alésage fileté (8; 29),
**caractérisé en ce que**
C) le dispositif comprend de plus comme moyen de fixation de l'os (12; 14) au moins une vis pour os (12) dotée d'une tête de vis (16) et d'une tige de vis (18) dotée d'un filet (19); et
D) la tête (16) de la vis présente un filet (17) dont le pas est plus petit que celui du filet (19) de la tige (18) de la vis.

2. Dispositif selon la revendication 1, **caractérisé en ce que** la tête (10) du clou médullaire est configurée comme élément séparé qui peut être placé sur l'extrémité proximale (3) du clou médullaire (1) et qui peut y être fixé de manière libérable.

3. Dispositif selon la revendication 1, **caractérisé en ce que** la tête (10) du clou médullaire est configurée d'un seul tenant avec le clou médullaire (1).

4. Dispositif selon l'une des revendications 1 à 3, **caractérisé en ce que** le clou médullaire (1) présente dans la région de l'extrémité distale (2) au moins un alésage (6) qui s'étend transversalement par rapport à l'axe central (4).

5. Dispositif selon l'une des revendication 1 à 4, **caractérisé en ce que** le clou médullaire (1) présente dans la région de l'extrémité proximale (3) au moins un alésage (7) qui s'étend transversalement par rapport à l'axe central (4).

6. Dispositif selon l'une des revendications 1 à 5, **caractérisé en ce que** la tête (10) du clou médullaire présente au moins trois alésages (11).

7. Dispositif selon la revendication 6, **caractérisé en ce que** les alésages (11) présentent des axes divergents.

8. Dispositif selon la revendication 6, **caractérisé en ce que** les alésages (11) présentent des axes parallèles.

9. Dispositif selon l'une des revendications 1 à 8, **caractérisé en ce que** l'alésage (11) de la tête (10) du clou médullaire présente un filet (15).

10. Dispositif selon la revendication 1, **caractérisé en ce que** le moyen (12; 14) de fixation de l'os est une vis pour os (12) qui possède une tête de vis (16) et une tige de vis (18) avec un filet (19), la vis pour os (12) étant de préférence traversée par un canal central (22).

11. Dispositif selon la revendication 1, **caractérisé en ce qu'**une partie de tige (21) non filetée est prévue entre les deux filets (17; 19).

12. Dispositif selon l'une des revendications 1 à 11, **caractérisé en ce que** la pointe (20) de la vis pour os (12) est autoforante ou autotaraudeuse.

13. Dispositif selon l'une des revendications 1 à 12, **caractérisé en ce que** le moyen (12; 14) de fixation de l'os est une lame (14) ou une plaque qui sont de préférence fendues ou alésées avec filetage, pour la réception de boulons ou de vis.

14. Dispositif selon l'une des revendications 1 à 13, **caractérisé en ce qu'**un autre alésage fileté (29) est prévu dans la région de l'extrémité proximale (3).
